# EUROPEAN PATENT APPLICATION

(11) **EP 2 270 202 A1**
(43) Date of publication of application: **05.01.2011**
(21) Application number: 09008732.1
(22) Date of filing: 03.07.2009
(51) Int. Cl.: C12Q 1/68

(54) **Mycobacterial detection**

(71) Applicant: Ikonomopoulos, John, 152 34 Halandri (GR)
(72) Inventor: Gazouli, Maria, 11633 Pagrati Athens (GR); Liandris, Emmanouil, 11361 Athens (GR); Ikonomopoulos, John, 15234 Halandri (GR)
(74) Representative: Malamis, Alkisti-Irene

(57) **Abstract**

The invention concerns mycobacteria-specific oligonucleotides sequences whose physico-chemical characteristics will be adequately variable to allow their further conjugation to quantum dots (QDs) in a way that will secure optimum performance (for example in systems based on Quantum Dots (QDs).

To this goal we have constructed a set of oligonucleotide probes that bind selectively to highly conserved regions of the mycobacterial genome and can be conjugated cadmium selenite QDs, aiming to the simultaneous detection of several mycobacterial DNA targets without molecular amplification.

The use of quantum dots (QDs)-universal-probes allows rapid and direct detection collectively of the main mycobacterial pathogens [*Mycobacterium tuberculosis* complex (MTC), *M. avium* complex (MAV) and *M. avium* subsp *paratuberculosis* (MAP)] in clinical samples, in a way that is highly specific, very easy to perform, and requires minimum infrastructure and expertise.

SEQUENCE LISTING

<110>  Ikonomopoulos, John

<120>  MYCOBACTERIUM DETECTION

<130>  epo/ikonom/QDs

<160>  21

<170>  PatentIn version 3.5

<210>  1
<211>  30
<212>  DNA
<213>  Mycobacterium

<400>  1
aacaccgaag tgaagtcgtt cgtgcagaag                                        30

<210>  2
<211>  30
<212>  DNA
<213>  Mycobacterium

<400>  2
tggatagtgg ttgcgagcat ggggtgtggt                                        30

<210>  3
<211>  30
<212>  DNA
<213>  Mycobacterium

<400>  3
ccatcttggt ggtggggtgt ccagtccgtg                                        30

<210>  4
<211>  30
<212>  DNA
<213>  Mycobacterium

<400>  4
accgtgaggg tgaaaagtac cccgggaggg                                        30

<210>  5
<211>  29
<212>  DNA
<213>  Mycobacterium

<400>  5
tcggtgaagg tcagcgaaca aggcctggc                                         29

<210>  6
<211>  30
<212>  DNA
<213>  Mycobacterium avium subsp paratuberculosis

<400>  6
gccacaacca cctccgtaac cgtcattgtc                                        30

<210>  7
<211>  30

<212>  DNA
<213>  Mycobacterium avium subsp paratuberculosis

<400>  7
tgattgttgg gtgcagggtc gctctgatga                                        30


<210>  8
<211>  30
<212>  DNA
<213>  Mycobacterium avium subsp paratuberculosis

<400>  8
tcaagcaggc cgccgagacg atttatcagg                                        30


<210>  9
<211>  30
<212>  DNA
<213>  Mycobacterium avium subsp paratuberculosis

<400>  9
gaagggtgtt cggggccgtc gcttaggctt                                        30


<210>  10
<211>  30
<212>  DNA
<213>  Mycobacterium avium subsp paratuberculosis

<400>  10
gaatatgttg ttgccctgtc taattcgatc                                        30


<210>  11
<211>  30
<212>  DNA
<213>  Mycobacterium avium complex

<400>  11
cgtcagtgac caaagtgccg aaaccattgc                                        30


<210>  12
<211>  30
<212>  DNA
<213>  Mycobacterium avium complex

<400>  12
gagttcctcg taatcaccgg cggcaacatg                                        30


<210>  13
<211>  30
<212>  DNA
<213>  Mycobacterium avium complex

<400>  13
aagccgaggt ggtgtatgtg ccgggccgca                                        30


<210>  14
<211>  30
<212>  DNA
<213>  Mycobacterium avium complex

<400>  14
gccaactgcc caaggtcgaa acgatgctgc                                        30

<210>  15
<211>  30
<212>  DNA
<213>  Mycobacterium avium complex

<400>  15
aataccggat aggacctcaa gacgcatgtc                                        30


<210>  16
<211>  30
<212>  DNA
<213>  Mycobacterium tuberculosis complex

<400>  16
ggtttgcggt ggggtgtcga gtcgatctgc                                        30


<210>  17
<211>  30
<212>  DNA
<213>  Mycobacterium tuberculosis complex

<400>  17
acaccgcaga cgttgatcaa catccggccg                                        30


<210>  18
<211>  30
<212>  DNA
<213>  Mycobacterium tuberculosis complex

<400>  18
gagccgggtg catgacaaca aagttggcca                                        30


<210>  19
<211>  30
<212>  DNA
<213>  Mycobacterium tuberculosis complex

<400>  19
cgtaggtcat agcgtaggaa ggcgggaatg                                        30


<210>  20
<211>  30
<212>  DNA
<213>  Mycobacterium tuberculosis complex

<400>  20
tcgaactcga ggctgcctac tacgctcaac                                        30


<210>  21
<211>  30
<212>  DNA
<213>  Mycobacterium

<400>  21
attagtaccg tgagggaatg gtgaaaagta                                        30

## Description

### Field of the invention

The general technical field of the invention relates to mycobacterium detection.

### Background to the invention

Pathogens of the genus *Mycobacterium* are a major cause of morbidity and mortality in humans and animals worldwide. Tuberculosis results nowadays to millions of deaths or disabilities each year, especially in poorer areas of the planet according to WHO reports, and this more than 100 years after the first description by Koch of the bacteria responsible. The problem is exacerbated by the AIDS epidemic that increases disease incidence in developed countries too. (www. who.org/tb/en).

Concerning humans, in addition to tuberculosis, exposure to mycobacteria has also been linked to the pathogenesis of sarcoidosis and Crohn's disease that affect millions of people in Europe only. The disease threat posed by the pathogens of the genus *Mycobacterium* is not unique to humans. Non-human primates can be infected by *Mycobacterium tuberculosis,* while cattle and other ruminants are natural hosts of the closely-related species *M. bovis,* which causes substantial financial loss in many regions of the world. The *M. avium* complex affects birds and mammals, including both livestock and dogs. Finally, paratuberculosis is another chronic infectious disease caused by a member of *Mycobacterium* spp., namely *M. avium* subsp *paratuberculosis* (MAP) that affects mainly ruminants.

The diagnosis of the disease in humans and in animals is made difficult by the fastidious nature of *M. tuberculosis;* the culture of *M. tuberculosis* is expensive and time-consuming due to the fastidious nature of the bacterium. Development of PCR has permitted the reduction of costs, time and labour associated with the diagnosis of mycobacterial diseases. Molecular techniques can be directly applied to clinical specimens permitting identification of mycobacterial DNA in shorter time and diminishing the biological risk associated with the diagnosis of mycobacteria. The characterization of the whole genome of the major pathogenic mycobacteria has permitted the identification of genetic markers that allow for the specific identification at species or even at strain level.

Diagnostic investigation of mycobacterial infections is also hampered by the difficulty to detect in a specific manner low populations of mycobacteria, or the immunology markers associated with the infections they cause. The laboratory diagnostic investigation of mycobacterial diseases relies on light microscopy of hematoxylin-eosin (H&E) or Ziehl-Neelsen (ZN) stained sections, mycobacterial culture, ELISA test and DNA amplification techniques. Each of these methods has certain advantages and limitations but in general, those with high specificity and low minimum detection limit are usually complex and expensive. In most cases the reliable application of these diagnostic methods requires highly trained personnel and very often, dedicated equipment that can be of considerable cost. Therefore, the development of a new diagnostic assay that would be easily applicable even by non-specialized personnel, and would allow specific and sensitive detection and identification of mycobacteria directly from clinical samples without the need of high cost dedicated equipment, would definitely improve diagnostic investigation of mycobacterial infections.

### Summary of the invention

The invention concerns mycobacteria-specific oligonucleotides sequences whose physicochemical characteristics will be adequately variable to allow their further conjugation to quantum dots (QDs) in a way that will secure optimum performance (for example in systems based on Quantum Dots (QDs).

To this goal we have constructed a set of oligonucleotide probes that bind selectively to highly conserved regions of the mycobacterial genome and can be conjugated cadmium selenite QDs, aiming to the simultaneous detection of several mycobacterial DNA targets without molecular amplification.

The use of quantum dots (QDs)-universal-probes allows rapid and direct detection collectively of the main mycobacterial pathogens [*Mycobacterium tuberculosis* complex (MTC), *M. avium* complex (MAV) and *M. avium* subsp *paratuberculosis* (MAP)] in clinical samples, in a way that is highly specific, very easy to perform, and requires minimum infrastructure and expertise.

Accordingly the invention provides a method of determining the presence of mycobacterial polynucleotides in a sample comprising contacting the sample with one or more signal oligonucleotides and determining whether the oligonucleotides bind to mycobaterial polynucleotides in the sample, wherein:
- at least one of the signal oligonucleotides is attached to a quantum dot, and the signal oligonucleotide is chosen from any of the oligonucleotides shown in tables 2 to 5 or a sequence which is at least 90% homologous to any of the sequences shown in tables 2 to 5, and wherein optionally the determination of said binding is carried out by detection of a change in photoluminescence.

### Detailed description of the invention

With connection to the oligonucleotides aimed to be used for conjugation with gold QDs, these were designed in pairs that anneal to mycobacteria-specific target sites situated on both sides of a 80-100 nm long genomic region, so that the neighbouring QDs do not overlap and do not stand more than 0,4 - 1 nm (2-5 nucleotides) away from each other.

With connection to the oligonucleotides aimed to be used for conjugation with cadmium selenite QDs, these were designed to be 30 bases long, so that apart from specific they can have adequate ionic strength in order to sustain these QDs that because of their outer layer require highly solid conjugation.

The mycobacteria target sites for both applications were selected from the 16s-rRNA, 16s-23s ITS and gyraseB genes, using the Primer Premier software. At this stage the work involved selection of only those oligonucleotide candidates that did not produce secondary structures and retained minimum annealing tendency to each other, so that they could be used in combinations. The BLAST-N v. 2.2.6 tool (National Center for Biotechnology Information, www.ncbi.nlm.nih.gov) was used to confirm that none of the selected oligonucleotides recognized any registered DNA sequence other than those that were targetbased. In total, a set of 30 oligonucleotides (10 for cadmium selenite and 10 pairs for gold QDs) was finally constructed in this way. The specificity of the designed probes was further evaluated by Dot Blot Hybridization assays with a panel of DNA samples extracted from various mycobacterial species and other bacteria commonly found in clinical samples. The present invention discloses the construction of highly specific oligonucleotide probes that can be used for identification of mycobacteria, and at the same time for conjugation with cadmium selenite and gold QD, thus enabling multi-target detection and multi-labelling.

We list the oligonucleotides that have proved to be more reliable for detection of *Mycobacteria spp, Mycobacterium tuberculosis comples* (MTB), *M. avium complex* (MAC) and *M. avium subsp paratuberculosis* (MAP).

QDs are semiconductor nanocrystals whose photoluminescence emission wavelength is proportional to the size of the crystal. The emission spectra of the QDs are narrow which allows multiwavelength labeling and effectively multi-target labeling with different sizes of QDs with little overlap. Their outer surface can be readily conjugated to organic molecules, resulting in a spectrum of biologically compatible labels that are excited with a single wavelength (Bruchez et al., 1998, Chan and Nie, 1998). Their integration to diagnosis of mycobacteria could greatly improve the detection limit of the technique without the need of amplification of the bacterial DNA.

QDs are already integrated in research and routine diagnostic applications. (Parungo et al., 2005).targeting and imaging of cancerous cells has been used by several research groups both in vivo (Gao et al., 2004, Stroh et al., 2005) and in vitro (Tokumasu et al., 2005). Selective distraction of these cells has also been exploited and offers a fascinating prospect for treatment (Sonvico et al., 2005). Recently, QDs have been used for the detection of several different bacterial species providing also information on their viability (Kloepfer et al., 2003, Su and Li, 2004, Dwarakanath et al., 2004).

QDs seem to be very promising as highly sensitive biosensors, since they already constitute the basis of a very innovative diagnostic approach. The ability to detect very small amounts of the QDs can decrease dramatically the lower detection limit of the diagnostic assays in which they are incorporated, allowing direct, even in-field detection of microbial nucleotide sequences, without the need for amplification. QDs are very promising as they could be used for the simultaneous quantitative detection of different targets. A great number of samples can be processed at the same time while the use of portable devices could permit the in-field detection of the QDs.

It becomes evident that a method that will combine the advantages of the diagnostic tests mentioned above [i.e. sensitivity of PCR without its low NPV (QDs will be able to detect oligonucleotide and polypeptide markers in the same sample), quantifiable results of ReT-PCR without its high cost (QDs will be easily recycled and reused, they do not necessarily require specific equipment), detection of mycobacteria specific peptides (multi-focal binding and multi-labelling of QDs can increase sensitivity even of non-monoclonal antibodies)], will greatly improve diagnostic investigation of mycobacterial infections.

The problem arises in that biomolecules designated to be used with QDs should be designed in a way that secure specificity for what they will detect and will render them at the same time, suitable for QD-conjugation.

Aim of the study that led to the present invention was the development of a set of oligonucleotides that could both
- detect the major pathogenic mycobacteria in a sensitive and specific way and
- fulfill the requirements for the stable conjugation of these molecules to CdSe QDs With the use of the proposed oligonucleotides conjugated with QDs will take us a step further since it will be developed to allow in addition to the above, assessment of mycobacterial viability through detection of proteins produced exclusively by viable cells [this assessment currently relies on reverse transcriptase PCR or NASBA, both of which have failed to be used routinely mainly because of their low reliability, i.e. positive results do not always signify microbial death and negative results are often false due to RNA degradation (David Rodriguez Lazzaro et al., Mol Cel Probes 2004)].

In one embodiment of the method it is carried out to diagnose infection of an individual suspected to have a mycobacterial infection, wherein the individual is preferably human.

As part of the method signal oligonucleotide may be added to polynucleotides which have been separated from the rest of the sample by means of a capture oligonucleotide, wherein the capture oligonucleotide is optionally attached to a magnetic bead and the signal oligonucleotide is optionally attached to a quantum dot, and wherein optionally the capture oligonucleotide is able to bind by Watson-Crick base pairing to polynucleotide sequence in the sample. Said separation may be achieved by contacting the sample with the capture oligonucleotide, allowing the capture oligonucleotide to bind to polynucleotide in the sample by Watson-Crick base pairing and then separating the capture oligonucleotide bound to polynucleotide, wherein optionally the capture oligonucleotide is bound to a magnetic bead and the separating is by means of a magnetic field.

The signal oligonucleotide is preferably chosen from any of the oligonucleotides shown in tables 2 to 5 or it may be an oligonucleotide which has homology with any of these specific oligonucleotides.

In one embodiment the capture oligonucleotide is labeled with biotin. In this embodiment the capture oligonucleotide may be attached via the biotin to streptavidin, and preferably the streptavidin is bound to (present on) the surface of the magnetic beads.

In a preferred embodiment pairs of oligonucleotides are used in detection, wherein one or both of the pairs are bound to a metal nanoparticle, which is optionally a gold nanoparticle. The pairs of oligonucleotides are the same as or homologous to any of the pairs of oligonucleotides shown in Table 3 (pairs are shown in adjacent rows in the same colour).

In the method 2, 3, 4 or more signal peptides are used, optionally labeled with different quantum dots labels. The different quantum dots may each have a different emission spectrum.

Typically, a QD is a semiconductor nanocrystal, whose radii are smaller than the bulk exciton Bohr radius, which constitutes a class of materials intermediate between molecular and bulk forms of matter. QDs are typically formed from inorganic, crystalline semiconductive materials and have unique photophysical, photochemical, and nonlinear optical properties arising from quantum size effects.

A QD may exhibit a number of unique optical properties due to quantum confinement effects. For example, QDs possess strong luminescence, photostability against bleaching and physical environments such as pH and temperature, and optical tunability, overcoming many of the shortcomings apparent with organic fluorophores.

QDs may be formed from an inner core of one or more first semiconductor materials that optionally may be contained within an overcoating or "shell" of a second semiconductor material. A QD core surrounded by a semiconductor shell is referred to as a "core/shell" QD. The optional surrounding shell material will preferably have a bandgap energy that is larger than the bandgap energy of the core material and may be chosen to have an atomic spacing close to that of the core substrate. Suitable semiconductor materials for the core and/or the optional shell include, but are not limited to, the following: materials comprised of a first element selected from Groups 2 and 12 of the Periodic Table of the Elements and a second element selected from Group 16 (e.g., ZnS, ZnSe, ZnTe, CDs, CdSe, CdTe, HgS, HgSe, HgTe, MgS, MgSe, MgTe, CaS, CaSe, CaTe, SrS, SrSe, SrTe, BaS, BaSe, BaTe, and the like); materials comprised of a first element selected from Group 13 of the Periodic Table of the Elements and a second element selected from Group 15 (e.g., GaN, GaP, GaAs, GaSb, InN, InP, InAs, InSb, and the like); materials comprised of a Group 14 element (Ge, Si, and the like); materials such as PbS, PbSe and the like; and alloys and mixtures thereof. As used herein, all reference to the Periodic Table of the Elements and groups thereof is to the IUPAC system for numbering element groups, as set forth in the Handbook of Chemistry and Physics, 81 st Edition (CRC Press, 2000). QDs may be made using techniques known in the art. See, e.g., U.S. Pat. Nos. 6,048,616; 5,990,479; 5,690,807; 5,505,928; and 5,262,357. QDs used in the present disclosure may absorb a wide spectrum of light, and may be physically tuned with emission bandwidths in various wavelengths. See, e.g., Badolato, et al., Science 208:1158-61 (2005). For example, the emission bandwidth may be in the visible spectrum (e.g., from about 3.5 to 7.5 [mu]m), the visible-infrared spectrum (e.g., from about 0.1 to about 0.7 [mu]m), or in the near-infrared spectrum (e.g., from about 0.7 to about 2.5 [mu]m). QDs that emit energy in the visible range include, but are not limited to, CdS, CdSe, CdTe, ZnSe, ZnTe, GaP, and GaAs. QDs that emit energy in the blue to near-ultraviolet range include, but are not limited to, ZnS and GaN. QDs that emit energy in the near-infrared range include, but are not limited to, InP, InAs, InSb, PbS, and PbSe. QDs with emission spectra in the near- infrared spectrum may be particularly suited for probes used in certain imaging applications. For example, such QDs may be suited for in vivo imaging, among other things, due to tissue's high optical transmissivity in the near-infrared spectrum.

For attachment, the QD may comprise a functional group or attachment moiety. One example of such a QD that has a functional group or attachment moiety is a QD with a carboxylic acid terminated surface, such as those commercially available though, for example, Quantum Dot, Inc., Hayward, CA.

According to another embodiment, the present disclosure provides a system that comprises a QD/metal nanoparticle complex as described herein and a detector capable of detecting luminescence from a QD. To detect luminescence, the detector should be positioned in relation to the complex such that luminescence can be detected, and the detector should be adapted to detect luminescence. One example of a suitable detector is a fluorimeter. In some embodiments, the system may further comprise a source of electromagnetic radiation, for example, an excitation monochromator. An excitation monochromator may be used to excide the probe at a specific wavelength to ensure the quality of emission detection.

Homologues of oligonucleotide sequences are referred to herein. Such homologues typically have at least 70% homology, preferably at least 80, 90%, 95%, 97% or 99% homology, for example over a region of at least 15, 20, 30 or more contiguous nucleotides, or even across the entire length of the original sequence. The homology may be calculated on the basis of nucleotide identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two oligonucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by 1, 2, less than 5, less than 10 or less than 15 mutations (each of which may be a substitution, deletion or insertion of a nucleotide). These mutations may be measured across any of the regions mentioned above in relation to calculating homology.

All publications which are mentioned above and below are incorporated herein by reference.

The following Examples illustrate the invention.

### Example 1

### Materials and methods

### Target selection for the identification of Mycobacterium spp.

For the specific identification of bacteria belonging to the genus *Mycobacterium* there were selected 3 genomic regions with high homology between the species of the genus. These regions are: a) 16s-rRNA gene b) ITS 16s-rRNA 23s-rRNA and c) gyrase B.

### Design of oligonucletide probes to be conjugated to CdSe QDs

The deposited sequences in the Entrez Nuceotide Database of the above genes for the major pathogenic mycobacteria (accession numbers DQ445257, NC008769, NC008595, NC002944, CP000325) were aligned with the use of ClustalW software. The design of the probes was done using Primer Premier software and was based on matching regions of each target.

Probes to be conjugated to CdSe QDs were 30 bases long in order to increase specificity and permit a more stable bond of the probes to the target.

We have designed oligonucleotide probes to be conjugated to CdSe QDs All probes were checked for their specificity through BLAST-N v. 2.2.6 (National Center for Biotechnology Information, www.ncbi.nlm.nih.gov).

### Determination of probe specificity by Dot Blot Hybridization

Probes were labeled with DIG labeling kit 2 (Roche, Mannheim Germany) according to the instructions of the manufacturer and were used in dot blot assays. Briefly, for each probe 10ng of DNA isolated from different bacteria (negative controls) and mycobacteria (positive controls) (table 1) was denaturated at 95°C for 5 minutes and fixed on nitrocellulose membrane through heating (80°C for 2 h). the membrane was incubated in hybridization buffer at 55°C for 30 minutes after which the labeled probe was added and the membrane was incubated overnight at the melting temperature of the probe.n The membrane was washed twice with 100ml of 0,1%SDS, 2xSSC, and twice with 100 ml 0,1% SDS, 0,5xSSC.

The revelation of the hybridization was done with the use of DIG Luminescent Detection Kit (Roche, Mannheim Germany) according to the instructions of the manufacturer. Probes exhibiting low specificity were discarded and the remaining 15 probes (5 for QDs and 5 pairs for AuNPs) (table 2) were subjected to further dot blot hybridization experiments in order to optimize the hybridization conditions.

### Results

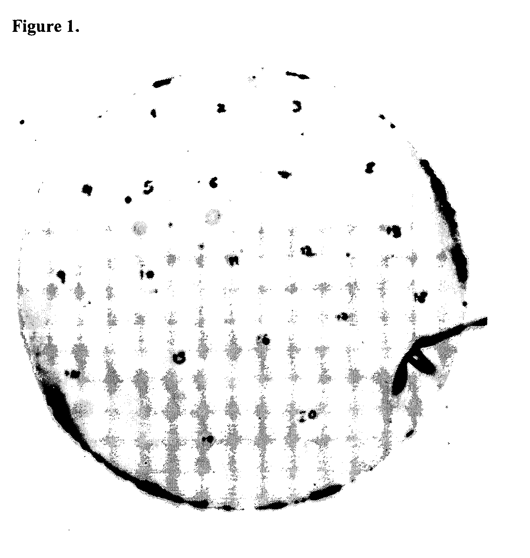

The nucleotidic sequence of the probes selected on the basis of their specificity for both CdSe QDs and AuNPs are presented in the tables. Figure 1 shows the results of a hybridization experiment.

**Table 1 Bacterial strains used as controls for the evaluation of the specificity of the oligonucleotide probes**

| **Bacterial species** | **Origin** |
|---|---|
| *M. tuberculosis* | University of Sassari, Dep. of Microbiology Italy |
| *M. tuberculosis* | University of Sassari, Dep. of Microbiology Italy |
| *M. tuerculosis* | Veterinary Research Institute, Brno, Czech Republic |
| *M. avium subsp paratuberculosis* | University of Sassari, Dep. of Microbiology Italy |
| *M. avium subsp paratuberculosis* | Veterinary Research Institute, Brno, Czech Republic |
| *M. avium subsp paratuberculosis* | Veterinary Research Institute, Brno, Czech Republic |
| *M. avium subsp avium* | University of Sassari, Dep. of Microbiology Italy |
| *M. avium subsp avium* | University of Sassari, Dep. of Microbiology Italy |
| *M. avium subsp avium* | Veterinary Research Institute, Brno, Czech Republic |
| *M. gordonae* | University of Sassari, Dep. of Microbiology Italy |
| *M gordonae* | Veterinary Research Institute, Brno, Czech Republic |
| *M. intracellulare* | University of Sassari, Dep. of Microbiology Italy |
| *M. intracellulare* | University of Sassari, Dep. of Microbiology Italy |
| *M. smegmatis* | University of Sassari, Dep. of Microbiology Italy |
| *M. smegmatis* | Veterinary Research Institute, Brno, Czech Republic |
| *Escherichia coli* | University of Sassari, Dep. of Microbiology Italy |
| *Escherichia coli* | University of Sassari, Dep. of Microbiology Italy |
| *Enterococcus fecalis* | University of Sassari, Dep. of Microbiology Italy |
| *Enterococcus fecalis* | University of Sassari, Dep. of Microbiology Italy |

**Table 2**

| Genus specific probes for quantum dot/CdSe conjugation | | | |
|---|---|---|---|
| Name | target | Seq 5'-3' | Tm |
| Myco1 | Gyrase B | AACACCGAAGTGAAGTCGTTCGTGCAGAAG | 67.5C |
| Myco2 | 16s rRNA | TGGATAGTGGTTGCGAGCATGGGGTGTGGT | 72.6C |
| Myco3 | 16s-23s ITS | CCATCTRGGTGGTGGGGTGTCCAGTCCGTG | 74.3C |
| Myco4 | 16s-23s ITS | ACCGTGAGGGTGAAAAGTACCCCGGGAGGG | 73.2C |
| Myco5 | Gyrase B | TCGGTGAAGGTCAGCGAACAAGGCCTGGC | 74.2C |

| MAP specific probes for quantum dot/CdSe conjugation | | | |
|---|---|---|---|
| MAP1 | IS900 | GCCACAACCACCTCCGTAACCGTCATTGTC | 70.3 |
| MAP2 | ISMAP02 | TGATTGTTGGGTGCAGGGTCGCTCTGATGA | 72.3 |
| MAP3 | IS900 | TCAAGCAGGCCGCCGAGACGATTTATCAGG | 72.9 |
| MAP4 | IS900 | GAAGGGTGTTCGGGGCCGTCGCTTAGGCTT | 74.6 |
| MAP5 | F57 | GAATATGTTGTTGCCCTGTCTAATTCGATC | 61.1 |

| MAV specific probes for quantum dot/CdSe conjugation | | | |
|---|---|---|---|
| MAV1 | IS 1245 | CGTCAGTGACCAAAGTGCCGAAACCATTGC | 71.2 |
| MAV2 | IS901 | GAGTTCCTCGTAATCACCGGCGGCAACATG | 73.2 |
| MAV3 | IS901 | AAGCCGAGGTGGTGTATGTGCCGGGCCGCA | 72.9 |
| MAV4 | IS1311 | GCCAACTGCCCAAGGTCGAAACGATGCTGC | 74.2 |
| MAV5 | 16s rRNA | AATACCGGATAGGACCTCAAGACGCATGTC | 65.6 |

| MTB specific probes for quantum dot/CdSe conjugation | | | |
|---|---|---|---|
| MTB1 | IS1611 | GGTTTGCGGTGGGGTGTCGAGTCGATCTGC | 74 |
| MTB2 | rpoB | ACACCGCAGACGTTGATCAACATCCGGCCG | 74.8 |
| MTB3 | 16s-23s | GAGCCGGGTGCATGACAACAAAGTTGGCCA | 73.8 |
| MTB4 | IS1611 | CGTAGGTCATAGCGTAGGAAGGCGGGAATG | 68.7 |
| MTB5 | IS1611 | TCGAACTCGAGGCTGCCTACTACGCTCAAC | 68.4 |

### Example 2

### Method Description

Magnetic beads coated with streptavidin were conjugated with biotinylated unique oligonucleotide specific for the *Mycobacterium* spp detection. The oligonucleotide sequence was: 5' - biotin-ATTAGTACCGTGAGGGAATGGTGAAAAGTA - 3' (capture oligonucleotide).

Quantum Dots purchased by Invitrogen were coated with streptavidin and conjugated with the appropriate biotinylated unique oligonucleotide (signal oligonucleotide) depending on the mycobacterium species that we want to detect each time (as presented in the following Tables)

**Table 3**

| For the detection of *Mycobacterium avium* spbs *paratuberculosis* | | |
|---|---|---|
| MAP 1 | IS900 | 5'-Biotin-GCCACAACCACCTCCGTAACCGTCATTGTC-3' |
| MAP2 | ISMAP02 | 5'-Biotin-TGATTGTTGGGTGCAGGGTCGCTCTGATGA-3' |
| MAP3 | IS900 | 5'-Biotin-TCAAGCAGGCCGCCGAGACGATTTATCAGG-3' |
| MAP4 | IS900 | 5'-Biotin-GAAGGGTGTTCGGGGCCGTCGCTTAGGCTT-3' |
| MAP5 | F57 | 5'-Biotin-GAATATGTTGTTGCCCTGTCTAATTCGATC-3' |

**Table 4**

| For the detection of *Mycobacterium avium complex* | | |
|---|---|---|
| MAV1 | IS 1245 | 5'-biotin-CGTCAGTGACCAAAGTGCCGAAACCATTGC-3' |
| MAV2 | IS901 | 5'-biotin-GAGTTCCTCGTAATCACCGGCGGCAACATG-3' |
| MAV3 | IS901 | 5'-biotin-AAGCCGAGGTGGTGTATGTGCCGGGCCGCA-3' |
| MAV4 | IS1311 | 5'-biotin-GCCAACTGCCCAAGGTCGAAACGATGCTGC-3' |
| MAV5 | 16s rRNA | 5'-biotin-AATACCGGATAGGACCTCAAGACGCATGTC-3' |

**Table 5**

| For the detection of *Mycobacterium tuberculsosis complex* | | |
|---|---|---|
| MTB1 | IS1611 | 5'-biotin-GGTTTGCGGTGGGGTGTCGAGTCGATCTGC-3' |
| MTB2 | rpoB | 5'-biotin-ACACCGCAGACGTTGATCAACATCCGGCCG-3' |
| MTB3 | 16s-23s | 5'-biotin-GAGCCGGGTGCATGACAACAAAGTTGGCCA-3' |
| MTB4 | IS 1611 | 5'-biotin-CGTAGGTCATAGCGTAGGAAGGCGGGAATG-3' |
| MTB5 | IS 1611 | 5'-biotin-TCGAACTCGAGGCTGCCTACTACGCTCAAC-3' |

DNA isolation from bacteria cultures and clinical samples was performed using commercial kits.

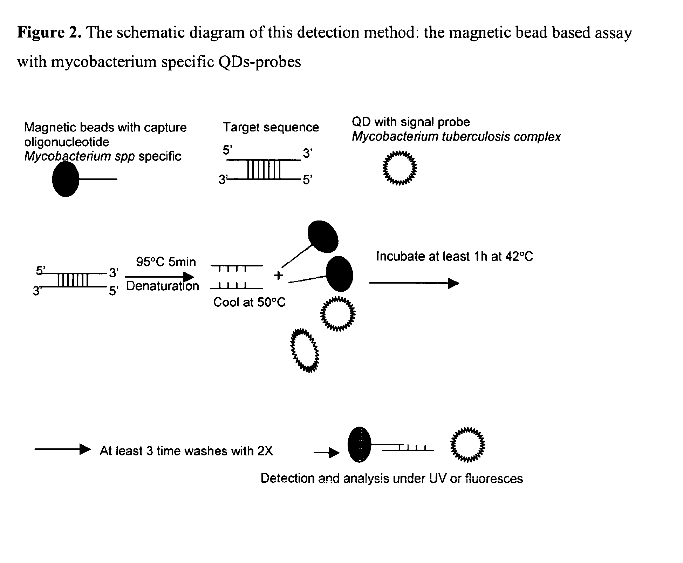

The schematic diagram of this detection method is shown in Figure 2.

Specifically, 20 µl HPLC water containing target DNA in a concentration approximately 30 ng/µl, was denaturated at 95°C for 5 min. When the solution cooled at 50°C, 20 µl of magnetic beads conjugated with the capture oligonucleotide and 30 µl of QD conjugated with the appropriate signal oligonucleotide were added in the solution containing the denatured DNA. The mixture was left for hybridization for at least one hour at 42°C. Then using a magnet apparatus to immobilized the magnetic beads the supernatant was removed and the beads were washed with 2X SSC [20X SSC: 0.3M trisodium citrate and 3.0M sodium chloride in high purity dH2O, pH 7.0] at least 3 times. Then the collected magnetic beads were resuspended in 20 µl 2XSSC and detected under UV light, or 5 µl of the solution was visualized under fluorescence microscope.

In positive samples the appropriate fluorescence color of QDs-probe was detected whereas in negative samples no fluorescence can be detected.

Our results indicate the QDs-probe method may offer fast and reliable mycobacterial DNA detection, without amplification of DNA. Furthermore, the QDs-probe assay reaction is performed in a single tube, which reduces carryover contamination. The method allows simple visual detection of the result that is made obvious by a sharp fluorescence sign, and it does not require dedicated high-cost equipment since for more precision it requires only a UV-spectrophotometer or a fluorescence microscope.

### Literature

Bruchez, M. Jr., Moronne, M., Gin, P., Weiss, S., Alivisatos, A.P., 1998. Semiconductor nanocrystals as fluorescent biological labels. Science. 281, 2013-2016.
Chan, W.C., Nie, S., 1998. Quantum dot bioconjugates for ultrasensitive nonisotopic detection. Science. 281, 2016-2018.
Cole, S.T., Brosch, R., Parkhill, J., Garnier, T., Churcher, C., Harris, D., Gordon, S.V., Eiglmeier, K., Gas, S., Barry, C.I., Tekaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K., Krogh, A., McLean, J., Moule, S., Murphy, L., Oliver, K., Osborne. J., Quail, M.A., Rajandream, M.A., Rogers, J., Rutter, S., Seeger, K., Skelton, J., Squares, R., Squares, S., Sulston, J.E., Taylor, K., Whitehead, S., Barrell, B.G., 1998. Deciphering the biology of Mycobacterium tuberculosis from the complete genome sequence. Nat. 393, 537-544.
Dwarakanath, S., Bruno, J.G., Shastry, A., Phillips, T., John, A.A., Kumar, A., Stephenson, L.D., 2005. Quantum dot-antibody and aptamer conjugates shift fluorescence upon binding bacteria. Biochem. Biophys. Res. Commun. 325, 739-743.
Gao, X., Cui, Y., Levenson, R.M., Chung, L.W., Nie, S., 2004. In vivo cancer targeting and imaging with semiconductor quantum dots. Nat. Biotechnol. 22, 969-976.
Kloepfer, J.A., Mielke, R.E., Wong, M.S., Nealson, K.H., Stucky, G., Nadeau, J.L., 2003. Quantum dots as strain- and metabolism-specific microbiological labels. Appl. Environ. Microbiol. 69, 4205-4213.
Parungo, C.P., Ohnishi, S., Kim, S.W., Kim, S., Laurence, R.G., Soltesz, E.G., Chen, F.Y., Colson, Y.L., Cohn, L.H., Bawendi, M.G., Frangioni, J.V., 2005. Intraoperative identification of esophageal sentinel lymph nodes with near-infrared fluorescence imaging. J. Thorac. Cardiovasc. Surg. 129, 844-850.
Rodríguez-Làzaro D, Lloyd J, Ikonomopoulos J, Pla M, Cook N., Unexpected detection of DNA by nucleic acid sequence-based amplification technique, Mol Cell Probes. 2004 Aug;18(4):251-3.
Sonvico, F., Dubernet, C., Colombo, P., Couvreur, P., 2005. Metallic colloid nanotechnology, applications in diagnosis and therapeutics. Curr. Pharm. Des. 11, 2095-2105.
Stroh, M., Zimmer, J.P., Duda, D.G., Levchenko, T.S., Cohen, K.S., Brown, E.B., Scadden, D.T., Torchilin, V.P., Bawendi, M.G., Fukumura, D., Jain, R.K., 2005. Quantum dots spectrally distinguish multiple species within the tumor milieu in vivo. Nat. Med. 11, 678-682.
Su, X.L., Li, Y., 2004. Quantum dot biolabeling coupled with immunomagnetic separation for detection of Escherichia coli O157:H7. Anal. Chem. 76, 4806-4810.
Tokumasu, F., Fairhurst, R.M., Ostera, G.R., Brittain, N.J., Hwang, J., Wellems, T.E., Dvorak, J.A., 2005. Band 3 modifications in Plasmodium falciparum-infected AA and CC erythrocytes assayed by autocorrelation analysis using quantum dots. J. Cell. Sci. 118, 1091-1098.

## Claims

1. A method of determining the presence of mycobacterial polynucleotides in a sample comprising contacting the sample with one or more signal oligonucleotides and determining whether the oligonucleotides bind to mycobaterial polynucleotides in the sample, wherein:
- at least one of the signal oligonucleotides is attached to a quantum dot, and
the signal oligonucleotide is chosen from any of the oligonucleotides shown in tables 2 to 5 or a sequence which is at least 90% homologous to any of the sequences shown in tables 2 to 5,
and wherein optionally the determination of said binding is carried out by detection of a change in photoluminescence.

2. A method according to claim 1 which is carried out to diagnose infection of an individual suspected to have a mycobacterial infection, wherein the individual is preferably human.

3. A method according to claim 1 or 2 wherein said contacting is performed by adding the signal oligonucleotide to polynucleotides which have been separated from the rest of the sample by means of a capture oligonucleotide, wherein the capture oligonucleotide is optionally attached to a magnetic bead and the signal oligonucleotide is optionally attached to a quantum dot, and wherein optionally the capture oligonucleotide is able to bind by Watson-Crick base pairing to polynucleotide sequence in the sample.

4. A method according to claim 3 wherein said separation is achieved by contacting the sample with the capture oligonucleotide, allowing the capture oligonucleotide to bind to polynucleotide in the sample by Watson-Crick base pairing and then separating the capture oligonucleotide bound to polynucleotide, wherein optionally the capture oligonucleotide is bound to a magnetic bead and the separating is by means of a magnetic field.

5. A method according to claim 3 or 4 wherein the signal oligonucleotide is chosen from any of the oligonucleotides shown in tables 2 to 5.

6. A method according to any one of claims 3 to 5 wherein the capture oligonucleotide is labeled with biotin which is bound to streptavidin present on the surface of the magnetic beads.

7. A method according to any one of the preceding claims wherein 2, 3, 4 or more signal peptides are used, optionally labeled with different quantum dots labels.

8. A method according to claim 7 in which the different quantum dots each have a different emission spectrum.

9. A method according to any one of the preceding claims in which the DNA in the sample has not been amplified and/or the sample is from an individual who does not have any symptoms of mycobacterial infection or disease.

10. A method according to any one of the preceding claims wherein all of the ligonucleotides shown in any one of tables 2 to 5, or homologues thereof, are used.

11. A kit for carrying out the method of any one of claims 1 to 10 comprising 1, 2 or 3 oligonucleotides as defined in claim 1.
